# EUROPEAN PATENT SPECIFICATION

(11) **EP 0 410 711 B1**
(45) Date of publication and mention of the grant of the patent: **15.12.1993**
(21) Application number: 90308131.3
(22) Date of filing: 25.07.1990
(51) Int. Cl.: A61L 27/00, A61L 31/00

(54) **Prosthesis**
Prothese
Prothèse

(30) Priority: 25.07.1989 US 385285; 06.03.1990 US 489373; 23.07.1990 US 557173
(43) Date of publication of application: 30.01.1991
(73) Proprietor: SMITH & NEPHEW RICHARDS, INC., Memphis, Tennessee 38116 (US)
(72) Inventor: Davidson, James A., Germantown, Tennessee 38138 (US)
(74) Representative: Gilholm, Stephen Philip

(56) References cited:
- EP-A- 0 159 410
- DE-A- 1 943 801
- DE-A- 2 811 603

## Description

This invention relates to prosthetic devices - more particularly, in one aspect to metallic implants which have load bearing surfaces such as those required for orthopaedic implants. Portions of such prostheses bear against surfaces which are subject to high rates of wear, for example the femoral head of a hip-stem prosthesis which engages a counter-bearing surface in an acetabular cup which is often made of a softer material such as ultra-high molecular weight polyethylene.

In another aspect, the invention also relates to the non-load bearing surfaces of such orthopaedic implants where a barrier is required between the metallic prosthesis and body tissue thereby preventing the release of metal ions and corrosion of the implant.

Orthopaedic implant materials must combine high strength and corrosion resistance with tissue compatibility. The longevity of the implant is of prime importance especially if the recipient is relatively young because it is desirable that the implant should function for the complete lifetime of a patient. Certain metal alloys have the required mechanical strength and biocompatibility and thus are ideal for use in the fabrication of prostheses. 316L stainless steel, chrome-cobalt-molybdenum alloys and more recently titanium alloys have proven to be the most suitable materials for the fabrication of load-bearing prostheses.

One of the variables affecting the longevity of load-bearing implants such as hip-joint implants is the rate of wear of the articulating surfaces and long-term effects of metal ion release. A typical hip-joint prosthesis includes a stem, a femoral head and an acetabular cup against which the femoral head articulates. Wear of either or both of the articulating surfaces results in an increasing level of wear particulates and "play" between the femoral head and the cup against which it articulates. Wear debris can contribute to adverse tissue reaction leading to bone resorption, and ultimately the joint must be replaced.

The rate of wear of the acetubular cup and the femoral head surfaces is dependent upon a number of factors which include the relative hardness and surface finish of the materials which constitute the femoral head and the acetabular cup, the frictional coefficient between the materials of the cup and head, the load applied and the stresses generated at the articulating surfaces. The most common material combinations currently used in the fabrication of hip-joint implants include femoral heads of cobalt or titanium alloys articulating against acetabular cups lined with organic polymer or composites of such polymers including, for instance, ultra-high molecular weight polyethylene (UHMWPE), and femoral heads of polished alumina in combination with acetabular cups lined with an orgnaic polymer or composite or made of polished alumina.

Of the factors which influence the rate of wear of conventional hip-joint implants, the most significant are patient weight and activity level. Additionally, heat which is generated by friction in the normal use of the implant as, for instance, in walking has been shown to cause accelerated creep and wear of the polyethylene cup. Furthermore, there is a correlation between the frictional moment which transfers torque loading to the cup and the frictional coefficient between the femoral head and the surface of the acetabular cup against which the head articulates. Cup torque has been associated with cup loosening. Thus, in general, the higher the level of torque generated, the greater the propensity for cup loosening. Ceramic bearing surfaces have been shown to produce significantly lower levels of frictional torque.

It is also noteworthy that two of the three commonly used hip-joint systems as indicated above include a metallic femoral head articulating against a UHMWPE liner inside the acetabular cup. UHMWPE, being a polymeric material, is more susceptable to creep when heated than the commonly used metal alloys or ceramics and is consequently more susceptable to wear than the alloys or ceramics.

It has also been found that metal prostheses are not completely inert in the body. The chemical interaction between the metals and the body fluids may have an undesirable effect on both the implant material and the biological host enviroment. The metal slowly corrodes and corrosion products in the form of metal ions may be released into the body. The rate of these processes are usally low enough not to compromise the performance of the prosthetic device, due to the presence of a passive, oxide or oxy-hydroxide, layer on the metal surface. However, when mechanical breakdown of passivity may occur by the partial or total removal of this protective layer during articulation against, for instance, UHMWPE, the corrosion processes may be significantly accelerated and the resultant increase in the metal ion release may be quite considerable.

Furthermore, the presence of third-body wear (cement or bone debris) accelerates this process and micro fretted metal particles can increase friction. Consequently, the UHMWPE liner inside the acetabular cup, against which the femoral head articulates, is subjected to accelerated levels of creep, wear and torque.

In US Patent No 4145764 it has been recognised that while metal prostheses have excellent mechanical strength they tend to corrode in the body by ionization. The affinity between ceramics and bone tissue was also recognised but it was noted that ceramic prostheses are weak on impact resistance. It was therefore proposed plasma spray a metal prosthesis with a bonding agent which is thereafter covered with a porous cermaic coating which would allow the ingrowth of bone spicules into the pores. This combination, it was said, would provide both the mechanical strength of metals and the biocompatibility of ceramics.

US Patent 4345764 did not address the issue of friction or wear of orthopedic implant bearing surfaces but confined itself to the single issue of the biocompatibility of metal prostheses. Nor did it address the issue of dimensional changes that occur when applying a coating or the effect of these dimensional changes in the tightness of fit between the surfaces of an articulating joint prosthesis.

In addition, the application of ceramic coatings to metal substrates often results in non-uniform, poorly-bonded coatings which tend to crack due to the differences in thermal expansion between the ceramic and the underlying metal substrate. Furthermore, such coatings are relatively thick (50-300 microns) and since the bond between the metal and the ceramic coating is often weak there is always the risk of galling or separation of the ceramic coating.

US Patent 3677795 is directed to the application of a carbide coating over a metallic prosthetic device. This method of forming the carbide coating requires that the prosthesis be heated to temperatures of at least about 1350°C in a reaction chamber through which a hydrocarbon gas such as propane or butane flows. The method is said to produce a prosthetics device which has "excellent compatibility with body tissue and is non-thombogenic." However, the issues of friction, heating, creep and wear of orthopedic implant bearing surfaces, or changes induced in the mechanical properties of the underlying metal due to this high temperature treatment are not addressed.

US Patent 3643658 is directed to titanium implants coated with titanium oxide, nitride, carbide or carbonitride to prevent corrosion and abrasion of the implant. These coatings are also said to protect the titanium implant from fretting wear. The coatings vary in thickness from 0.08 µm to about 0.15 µm. Despite these teachings titanium oxide coatings are not as well attached, are not as dense and adherent, and are not effective as protective coatings to prevent metal ion release into the body. Titanium oxide forms naturally on titanium and titanium alloy in ambient conditions. This oxide film is thin (.5-7 nm) to a point where it is transparent to the naked eye and is similar to the protective passive oxide layers formed primarily from the chromium content in cobalt allys and stainless steels. Formation of these types of natural, passive oxide layers under ambient conditions or nitric acid passivation conditions (usually used for metal orthopaedic implants) can easily abrade off from motion and contact against surrounding material, even soft polymeric materials. Under these conditions, metal ions are released into the environment. For the case of titanium and titanium alloys, amorphous titanium monoxide (Ti0) forms at room temperature with small quantities of Ti₃0₅. The oxide is easily disturbed in a saline environment resulting in repassivation of an intermediate oxide 3Ti₂O₃·4TiO₂. Formation of the higher oxide, TiO₂ (anatase) and Ti₂O occur at higher oxidation temperatures. However, under fretting conditions (with adjacent bone, bearing against polyethylene and particularly against metal as in the case for bone screws in bone plates, etc.) all forms of normal passivated, and even high-temperature (350°C) surface anodized titanium oxide films provide little, if any, protection from spalling of the oxide and subsequent fretting of the metal substrate. Relatively thicker coatings using high current-density anodizing also provide little anti-fretting protection due to the poor adherent of the loose powdery films. In general, titanium oxide films are ineffective against fretting conditions because of their poor strength and attachment.

A totally inert, abrasion resistant monolithic ceramic may be ideal for eliminating fretting and metal ion release. For example, zirconium dioxide (ZrO₂) and alumina (Al₂O₃) have been shown to be highly inert, biocompatible implant materials. These ceramics have been in use recently as monolithinc alumina or zirconium dioxide femoral heads in total hip replacements. Both materials are hard, dense, biocompatible and sufficiently strong. Importantly, when polished, the ceramic bearing surface, articulating against ultra high molecular weight polyethylene (UHMWPE), not only significantly reduces the frictional moment against the UHMWPE cup but also greatly reduces the rate of wear of the UHMWPE. During articulation, no metal ions or micro-size fretting particulates from the ceramic are produced. Thus, these ceramics are advantageous over cobalt, stainless steel, and titanium alloy bearing surfaces. Micron-size metal fretting debris which occurs from metal bearing surfaces is osteolytic (can kill bone cells). However, monolithic ceramics are difficult and costly to manufacture, can crack (fragment) under extreme impact, and have a relatively high elastic modulus. Thus, the use of such ceramic materials in monolithic form is not practical in femoral components for total knee prostheses.

Currently used cobalt alloy knee femoral components have been used successfully for many years. However, measurable levels of potentially toxic metal ions and osteolytic micron fretting debris can be generated. Further, the frictional torque against UHMWPE tibial bearing surfaces and the wear rate of the UHMWPE surface is less than optimal. A ceramic femoral knee prosthesis would be expected to reduce wear and torque due to lower friction and would virtually eliminate metal ion release. However, a monolithic ceramic femoral knee component would be costly, difficult to manufacture because of the complex shape, and would still be highly susceptible to impact fracture due to the thin cross-section. The high modulus ceramic would also limit load transfer to the underlying bone. Such loading is imperative for maintaining viable, supportive bone in non-cemented implants. Additionally, a monolithic ceramic knee femoral component would be difficult to manufacture with a porous coating for bone ingrowth. Because limited bone ingrowth is observed with existing porous metal coatings, a thin ceramic coating on a porous metal surface is expected to offer an improvement. To improve load transfer the lower modulus titanium alloys, protected from abrasion using ion-implantation techniques, have been tried as knee femoral components. However, such coatings are extremely thin (about 0.5µm) and exhibit only an temporary improvement in metal ion release over titanium alloy fretting against UHMWPE. Moreover, ion-implantation does not eliminate production of micron size fretting debris from the surface particularly in the presence of third body debris, such as bone cement particulates, and does not improve wear of the UHMWPE.

Particulates of metal fretted from metal bearing surfaces can retard growth of bone cells. Polyethylene wear debris produces a severe inflammatory response and a proliferation of giant cells and enzyme response which can lead to loosening and eventual revision of the implant. Thus, the optimum knee or hip joint femoral component is one in which an inert, low friction, low wear (of the UHMWPE) ceramic bearing surface is present, but without the fracture susceptibility and stiffness of monolithic ceramic.

Zirconium and zirconium alloys offer the additional advantage over existing cobalt, stainless steel and titanium alloys in that the elastic modulus is lower (about 90G Pa) and can thus more effectively transfer load to adjacent bone.

There exists a need for a metal alloy-based orthopedic implant having low friction, highly wear resistant load bearing surfaces which may be implanted for the lifetime of the recipient. There also exists a need for a metal alloy-based orthopedic implant that is not prone to corrosion by the action of body fluids so that it is biocompatible and stable over the lifetime of the recipient.

In accordance with the present invention there is provided a prosthesis having a prosthesis body comprising zirconium or a zirconium alloy, the body having a surface region comprising an oxide or nitride of zirconium, wherein the oxygen or nitrogen concentration in the surface region decreases from a maximum at its outer surface to a minimum at the inner part of the region.

The zirconium oxide or nitride coating provides the invention prosthesis with a thin, dense, low friction, wear resistant, biocompatible surface idealy suited for use cn articulating surfaces of joint prostheses wherein a surface or surfaces of the joint articulates, translates or rotates against mating joint surfaces. The zirconium oxide or nitride coating may therefore be usefully employed on the femoral heads or inside (metal shell) surfaces of acetabular cups of hip-joint implants on the articulating surfaces of other types of prostheses, such as femoral and tibial (meniscal bearing) surfaces of knee joints.

According to an aspect of the invention there is provided a prosthesis for implantation in a patient, comprising:
a) a prothesis body formed of zirconium or zirconium alloy, at least a portion of which is adapted to penetrate and engage body tissues, and
b) a thin coating comprising blue-black or black zirconium oxide or zirconium nitride directly on at least the portion of the prosthesis body adapted to penetrate and engage body tissues for preventing metal ion release from the coated portion of the prosthesis body.

In accordance with another aspect of the invention there is provided a prosthesis for implantation in a patient, comprising:
a) a prosthesis body formed of zirconium or zirconium alloy including an implant portion for penetrating and engaging body tissues, of the patient;
b) a bearing surface on the prosthesis body, the bearing surface being sized and shaped to engage a bearing surface on another prosthesis portion; and
c) a thin coating of zirconium oxide directly on at least the bearing surface of the prosthesis body for making the bearing surface inert, more wear resistant and of a lower coefficient of friction.

According to a further aspect of the invention there is also provided a prosthesis for implantation in a patient, comprising:
a) a prothesis body formed of zirconium or zirconium alloy including an implant portion for penetrating and engaging body tissues, of the patient;
b) a bearing surface on the prosthesis body, the bearing surface being sized and shaped to engage a bearing surface on another prosthesis portion; and
c) a thin coating of zirconium nitride directly on at least the bearing surface of the prosthesis body for making the bearing surface inert, more wear resistant and of a lower coefficient of friction.

By the term "coating" it is meant the covering of material over the prosthesis body. The materials per se need not be coated directly onto the prosthesis body but can also be formed in situ by reaction of the metal of the body at the region of the surface with an oxygen or nitrogen donor or with the elements themselves. Although these materials at the surface region may be in the form of discrete or homogenous layers or films the term "coating" also encompasses diffuse coverings. For example, the material at the outer surface of the surface region may comprise stoichiometric zirconium oxide. However, the oxygen concentration decreases with increasing depth from the outer surface. The hardness of the oxygen alloy below the zirconium oxide surface is approximately equal to that of the oxide itself and gradually diminishes towards the interior of the prosthesis body as the level of oxygen decreases.

Similar effects may be produced under nitriding conditions although the corresponding nitriding reactions are thermodynamically far less favourable.

When a zirconium oxide or nitride-coated joint surface is employed in a manner wherein it articulates or rotates against a non-metallic or non-zirconium oxide or nitride-coated surface, the low friction characteristic of the coating causes reduced friction, wear, and heat generation in comparison to that of prior art prostheses. This reduced heat generation results in a lowered tendency for the non-metallic or non-zirconium oxide or nitride coating bearing surface to experience creep and torque so that the useful life of the opposing surface is enhanced. Thus, for instance, where the zirconium oxide or nitride coated femoral head of a hip joint implant articulates against an opposing ultra-high molecular weight polyethylene (UHMWPE) surface liner of an acetabular cup, friction and wear is reduced so that the uhmwpe is subjected to lower levels or torque, wear and heat generation. Consequently the UHMWPE experiences lowered levels of creep and cup loosening is reduced resulting in an enhancement of the life of the liner and the prosthesis.

The zirconium oxide or nitride coating of the subject invention is also useful in providing a biocompatible, inert ceramic barrier between the zirconium-containing metal or alloy-based prothesis and body fluids. Thus, since the zirconium oxide or nitride surface is not prone to dissolution and wear induced corrosion, both the life span and the biocompatibility of the prosthesis are enhanced.

Additionally, the preferred natural in situ formation of a zirconium oxide coating from the presence of zirconium in the substrate metal involves oxygen diffusion into the metal substrate below the oxide coating. Oxygen, an alloying constituent in zirconium, increases the strength of the metal substrate, particularly the fatigue strength. Resistance to fatigue loading is paramount in many orthopedic implant applications such as the hip stem, and femoral and tibial knee components. Thus, not only does the formation of the zirconium oxide coating improve wear, friction and corrosion resistance, it also improves the mechanical integrity of the implant device from a strength standpoint.

Figure 1 is a schematic diagram depicting a hip joint prosthesis is position.

Figure 2 is a schematic diagram showing a typical hip joint prosthesis.

Figure 3 is a schematic diagram of a knee joint prosthesis in place.

Figure 4 is a schematic diagram of the parts of a typical knee joint.

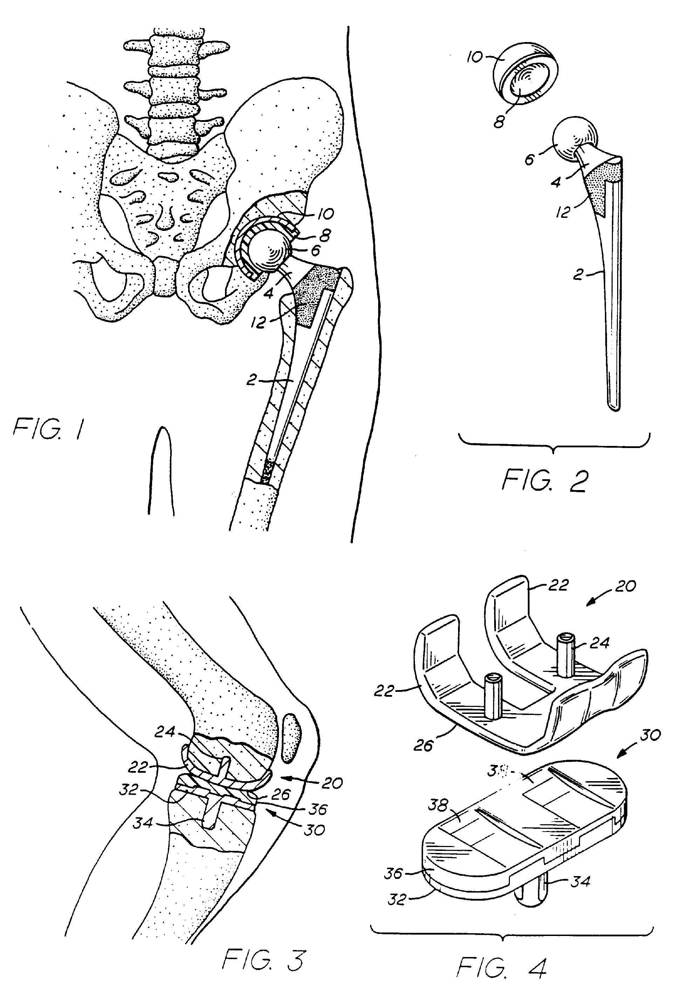

One aspect of the invention is to provide low friction, wear resistant coatings on the articulating surfaces of prosthetic devices. Illustrative examples of such articulating surfaces are shown in the schematic diagrams, figures 1-4.

A typical hip joint assembly is shown in situ in figure 1. The hip joint stem 2 fits into the femur while the femoral head 6 of the prosthesis fits into and articulates against the inner lining 8 of an acetabular cup 10 which is turn is affixed to the pelvis as shown in Fig. 1. A porous metal bead or wire mesh coating 12 may be incorporated to allow stabilization of the implant by ingrowth of surrounding tissue into the porous coating. Similarly, such a coating can also be applied to the acetabular component. The femoral head 6 may be an integral part of the hip joint stem 2 or may be a separate component mounted upon a conical taper at the end of the neck 4 of the hip joint prosthesis. This allows the fabrication of a prosthesis having a metallic stem and neck but a femoral head of some other material, such as ceramic. This methods of construction is often desirable because ceramics have been found to generate less frictional torque and wear when articulating against the UHMWPE then alumina. Regardless of the materials, however, the femoral head articulates against the inner surface of the acetabular cup thereby causing wear and, in the long term, this may necessitate prosthesis replacement. This is especially the case where the femoral head is of metal and the acetabular cup is lined with an organic polymer or composite thereof. While these polymeric surfaces provide good, relatively low friction surfaces and are biocompatible, they are as explained above, subject to wear and accelerated creep due to the frictional heat and torque to which they are subjected during ordinary use.

A typical knee joint prosthesis is shown in situ in figure 3. The knee joint includes a femoral component 20 and a tibial component 30. The femoral component includes condyles 22 which provide the articulating surface of the femoral component and pegs 24 for affixing the femoral component to the femur. The tibial component 30 includes a tibial base 32 with a peg 34 for mounting the tibial base onto the tibia. A tibial platform 36 is mounted atop the tibial base 32 and is supplied with grooves 38 similar to the shape of the condyles 22. The bottom surfaces of the condyles 26 contact the tibial platform's grooves 38 so that the condyles articulate within these grooves against the tibial platform. While condyles are typically fabricated of metals, the tibial platform may be made from an organic polymer or a polymer based composite. Thus, the hard metallic condyle surfaces 26 would articulate against a relatively softer organic composition. As previously explained, this may result in wear of the organic material, ie. The tibial platform necessitating the replacement of the prosthesis. Meniscal bearing designs of the tibial component can allow for greater contact area without significant sacrifice of knee constraint, thereby reducing wear. Ceramic coatings of both the knee femoral component and the miniscal bearing features in the tibia will further reduce wear of the polyethylene. As in the case of the hip joint, a porous bead or wire mesh coating can also be applied to either the tibial or femoral components of the knee or both.

The invention provides blue-black or black zirconium oxide or zirconium nitride coated orthopedic implants or prostheses fabricated of zirconium or zirconium containing metal alloys or a thin coating of zirconium or zirconium alloys on conventional orthopedic implant materials. In order to form continuous and useful zirconium oxide or nitride coatings over the desired surface of the metal alloy prosthesis substrate, the prothesis substrate should be zirconium per se and should contain from at least about 80 wt.% preferably at least about 95 wt.% zirconium. Oxygen, niobium, tantalum and titanium may be included as common alloying elements in the alloy optionally in the presence of hafnium. Yttrium may also be alloyed with the zirconium to enhance the formation of a tougher, yttria-stabilized zirconium oxide coating during the oxidation of the alloy. While such zirconium containing alloys may be custom formulated by conventional methods known in the art of metallurgy, a number of suitable alloys are commercially available. These commercial alloys include among others zircadyne 705, zircadyne 702, and zircalloy.

The base zirconium containing metal alloys are cast or machined from wrought metal stock by conventional methods to the shape and size desired to obtain a prosthesis substrate. The substrate is then subjected to process conditions which cause the natural (in situ) formation of a tightly adhered, diffusion-bonded coating of zirconium oxide on its surface. The process conditions include, for instance, air, steam, or water oxidation or oxidation in a salt bath. These processes ideally provide a thin, hard, dense, blue-black, low friction wear-resistant zirconium oxide film or coating of thickness typically less than several µm (10⁻⁶ meters) on the surface of the prosthesis substrate. Below this coating, diffused oxygen from the oxidation process increases the hardness and strength of the underlying substrate metal.

Unlike the prior art titanium oxides of, for example, steinemann's US patent 3643658, the oxygen supplied to form the blue-black or black zirconium oxide coatings of the invention is a beneficial alloying component which improves the fatigue strength of the underlying zirconium metal thereby increasing the potential life of the prosthesis. In contrast, oxygen in titanium alloys tends to stabilise the lower strength, α-phase which significantly reduces the metal's fatigue strength.

The air, steam and water oxidation processes are described for example in US patent 2987532. The air oxidation process provides a firmly adherent black or blue-black layer of zirconium oxide of highly oriented monoclinic crystalline form. If the oxidation process is continued to excess, the coating will whiten and separate from the metal substrate. The oxidation step may be conducted in either air, steam or hot water. For convenience the metal prosthesis substrate may be placed in a furnace having an oxygen-containing atmosphere (such as air) and typically heated at 370-595°C (700-1100°F) up to about 6 hours. However, other combinations of temperature and time are possible. When higher temperatures are employed, the oxidation time should be reduced to avoid microcracking or the formation of the white oxide.

It is preferred that a blue-black zirconium oxide layer ranging in thickness from 1 to 5 µm should be formed. For example, furnace air oxidiation at 538°C (1000°F) for 3 hours will form an oxide coating on Zircadyne 705 about 3-4 µm thick. Longer oxidation times and higher oxidation temperatures will increase this thickness, but may compromise coating integrity. For example, one hour at 705°C (1300°f) will form an oxide coating about 14 µm in thickness, while 21 hours at 538°C (1000°f) will form an oxide coating thickness of about 9 µm. Of course, because only a thin oxide is necessary on the surface, only very small dimensional changes, typically less than 10 µm, for example 1 to 5 microns, over the thickness of the prosthesis, will result. In general, thinner coatings (1-4 µm) have better attachment strength.

The thickness of the blue-black or black zirconium oxide coatings on the invention prostheses provides a further distinction between the invention and the titanium oxide coatings of US patent 3643658 to steinemann. Titanium oxide films, whether prepared by high temperature (350°C) oxidation or high current density anodizing, are thin, powdery and loosely adherent. Consequently, these films can be more easily removed under fretting conditions in vivo exposing metal surface to bodily fluids with resulting metal ion release into the body tissue. The thicker, crystalline, more tightly adherent blue-black or black zirconium oxide films, by contrast, do not readily spall or separate from the alloy substrate. It is speculated that the diffusion of oxygen into the zirconium alloy provides a natural interlayer to which the zirconium oxide can adhere readily and tightly. Consequently, these zirconium oxide coatings provide excellent protection against fretting corrosion by bodily fluids. Thus an additional advantage of the in situ formed coatings or regions of zirconium oxide is their ability to maintain protective barriers to fretting of porous coatings againt bone or fretting from adjacent prothesis components and materials such as modular components, bone plates and bone screws. In the light of this finding components such as modular components, bone plates and bone screws made of zirconium or zirconium alloy and having the in-situ formed oxidic or nitridic coatings or regions would provide improved fretting performance.

One of the salt-bath methods that may be used to apply the zirconium oxide coatings to the metal alloy prosthesis, is the method of US Patent 4671824. The salt-bath method provides a similar, slightly more abrasion resistant blue-black or black zirconium oxide coating. The method requires the presence of an oxidation compound capable of oxidizing zirconium in a molten salt bath. The molten salts include chlorides, nitrates, cyanides and the like. The oxidation compound, sodium carbonate, is present in small quantities, up to about 5wt. %. The addition of sodium carbonate lowers the melting point of the salt. As in air oxidation, the rate of oxidation is proportional to the temperature of the molten salt bath. A preferred range of 550°-800°c (1022°-1470°f) is disclosed in US Patent No. 4671824. However, the lower oxygen levels in the bath produce thinner coatings than for furnace air oxidation at the same time and temperatures. A salt bath treatment at 1290°f for four hours produces an oxide coating thickness of roughly 7 µm.

Whether air oxidation in a furnace or salt-bath oxidation is used, the zirconium oxide coatings are quite similar in hardness. For example, if the surface of a wrought zircadyne 705 (Zr, 2-3 wt.% Nb) prosthesis substrate is oxidised, the hardness of the surface shows a dramatic increase over the 200 Diamone Pyramid Hardness (DPH) of the original metal surface. The surface hardness of the blue-black zirconium oxide surface following oxidation by either the salt bath or air oxidation process is approximately 1700-2000 (DPH).

These diffusion-bonded, low friction, highly wear resistant zirconium oxide coatings are applied to the surfaces of orthopedic implants subject to conditions of wear or fretting. Such surfaces include the articulating surfaces of knee joints, elbows and hip joints. Additionally, the porous coated regions, modular components, bone plates, bone screws and other trauma devices subjected to fretting conditions can be included. As mentioned before, in the case of hip joints, the femoral head and stem are typically fabricated of metal alloys while the acetabular cup may be fabricated from ceramics, metals or organic polymer-lined metals or ceramics.

When the zirconium oxide coated femoral head is used in conjunction with any of these acetabular cups, the coefficient of friction between the femoral head and the innner surface of the cup is reduced so that less heat and torque is generated and less wear of the mating bearing surface results. This reduction in heat generation, frictional torque, and wear is particularly Important in the case of acetabular cups lined with organic polymers or composites of such polymers. Organics polymers, such as UHMWPE, exhibit rapidly increased rates of creep when subjected to heat with consequent deleterious effect on the life span of the liner. Wear debris of the polymer leads to adverse tissue response and loosening of the device. Thus, not only does the zirconium oxide coating serve to protect the prosthesis substrate to which it is applied and increase its mechanical strength properties but, as a result of its low friction surface, it also protects those surfaces against which it is in operable contact and consequently enhances the performance and life of the prosthesis.

The usefulness of zirconium oxide coated prosthesis is not limited to load bearing prostheses, especially joints, where a high rate of wear may be encountered. Because the zirconium oxide coating is firmly bonded to the zirconium alloy prosthesis substrate, it provides a barrier between the body fluids and the zirconium alloy metal thereby preventing the corrosion or fretting of the alloy by the process of ionization and its associated metal ion release.

Oxygen diffusion into the metal substrate during oxidation also increase the stength of the metal. Consequently, a zirconium oxide coated prosthesis may be expected to have a greater useful service life.

Zirconium or zirconium alloy can also be used to provide a porous bead or wire mesh surface to which surrounding bone or other tissue may integrate to stabilize the prosthesis. These porous coatings can be treated simultaneously by the oxidation treatment in a manner similar to the oxidation of the base prosthesis for the elimination or reduction of metal ion release. Furthermore, zirconium or zirconium alloy can also be used for fretting-susceptible devices such as bone plates and screws, or as a surface layer applied over conventional implant materials prior to in situ oxidation and formation of the zirconium oxide coating.

In situ oxidation is the preferred method for producing the invention oxide coatings because it allows oxygen diffusion into the metal substrate thereby allowing the formation of a tightly adherent oxide coating while also strengthening the zirconium metal. Other techniques, such as depositing an oxide coating on the prosthesis substrate may also be used but the coatings produced may not be as effective as those produced by the in situ process. Thus, chemical or physical vapour deposition methods may be used, especially those using an ion-assisted deposition method.

While the above discussion has dealt mainly with blue-black or black zirconium oxide coatings on prostheses, zirconium nitride coatings are also effective in reducing wear on opposing surfaces and preventing corrosion of the underlying substrate by bodily fluids.

Even though air contains about four times as much nitrogen as oxygen, when zirconium or a zirconium alloy is heated is air as described above, the oxide coating is formed in preference to the nitride coating. This is because thermodynamically oxidation is favoured over nitridation under these conditions. Thus, to form a nitride coating the equilibrium must be forced into favouring the nitride reaction. This is achieved by elimination of oxygen and using a nitrogen or nitrogen-forming atmosphere such as ammonia atmosphere instead of air or oxygen when a gaseous environment (analogous to "air oxidation") is used. The nitriding conditions would be those conventionally employed for nitriding.

When a salt bath method is used to produce a nitride coating, then the oxygen-donor salts should be replaced with nitrogen-donor salts, such as, for instance cyanide salts. Upon such substitution, a nitride coating may be obtained under similar conditions to those needed for obtaining an oxide coating. Such modifications as are necessary, may be readily determined by those of ordinary skill in the art.

Alternatively, the zirconium nitride may be deposited onto the zirconium or zirconium alloy surface via standard physical or chemical vapour deposition methods, including those using an ion-assisted deposition method. It is preferred that the physical or chemical vapour deposition methods be carried out in an oxygen-free environment. Techniques for producing such an environment are known in the art, for instance the bulk of the oxygen may be removed by evacuation of the chamber and the residual oxygen may be removed with an oxygen getter.

When the zirconium or zirconium alloy is provided with a zirconium porous bead or zirconium wire mesh surface, then this surface layer can also be coated with zirconium nitride to provide protection against metal ionizationin the body.

## Claims

1. A prosthesis having a prosthesis body portion comprising zirconium or a zirconium alloy, the body portion having a surface region comprising an oxide or nitride of zirconium, wherein the oxygen or nitrogen concentration in the surface region decreases from a maximum at its outer surface to a minimum at the inner part of the region.

2. A prosthesis according to claim 1 wherein the surface region of the prosthesis body portion is a bearing surface and is adapted to engage another prothesis body.

3. A prosthesis according to claim 1 or claim 2 wherein the prosthesis body portion is adapted to penetrate and engage body tissue.

4. A prosthesis according to claim 3 wherein the prosthesis body portion is a screw.

5. A prosthesis according to claim 1 or claim 2 wherein the posthesis body portion is adapted to be attached to body tissue or to another portion of a prosthesis.

6. A prosthesis according to claim 5 wherein the prosthesis body portion is a bone plat.

7. A prosthesis according to any one of the preceding claims in which the prosthesis body portion is a modular component.

8. A prosthesis for implantation in a patient, comprising:
a) a prothesis body formed of zirconium or zirconium alloy, at least a portion of which is adapted to penetrate and engage body tissues, and
b) a thin coating comprising blue-black or black zirconium oxide or zirconium nitride directly on at least the portion of the prosthesis body adapted to penetrate and engage body tissues for preventing metal ion release from the coated portion of the prosthesis body.

9. A prosthesis according to any one of the preceding claims, wherein the body portion further comprises an irregular surface structure adapted to accommodate tissue ingrowth on the portion of the prosthesis body adapted to penetrate and engage body tissue.

10. A prosthesis according to claim 9, wherein the irregular surface structure is formed of zirconium or zirconium alloy beads connected to the outer surface of the prosthesis body.

11. A prosthesis according to claim 9, wherein the irregular surface structure is formed of a zirconium or zirconium alloy wire mesh connected to the outer surface of the prosthesis body.

12. A prosthesis according to any one of the preceding claims wherein said surface region extends over the entire prosthesis.

13. A prosthesis according to any one of the preceding claims wherein the surface region is less than 10 µm thick.

14. A prosthesis for implantation in a patient, comprising:
a) a prosthesis body formed of zirconium or zirconium alloy including an implant portion for penetrating and engaging body tissues, of the patient;
b) a bearing surface on the prosthesis body, the bearing surface being sized and shaped to engage a bearing surface on another prosthesis portion; and
c) a thin coating of zirconium oxide directly on at least the bearing surface of the prosthesis body for making the bearing surface inert, more wear resistant and of a lower coefficient of friction.

15. A prosthesis for implantation in a patient, comprising:
a) a prothesis body formed of zirconium or zirconium alloy including an implant portion for penetrating and engaging body tissues, of the patient;
b) a bearing surface on the prosthesis body, the bearing surface being sized and shaped to engage a bearing surface on another prosthesis portion; and
c) a thin coating of zirconium nitride directly on at least the bearing surface of the prosthesis body for making the bearing surface inert, more wear resistant and of a lower coefficient of friction.

16. A prosthesis according to any one of the preceding claims wherein the prosthesis body is the portion of a hip prosthesis adapted to be implanted in a femur.

17. A prosthesis according to any one of claims 1 to 11 wherein the bearing surface of the prosthesis body includes a head portion adapted to articulate with an acetabular cup formed of an organic polymer or polymer-based composite.

18. A prosthesis according to any one of claims 1 to 11 wherein the prosthesis body is the portion of a knee prosthesis adapted to be implanted in a femur or tibia.

19. A prosthesis according to any one of claims 1 to 11 wherein the bearing surface of the prosthesis body is at least one condyle portion adapted to articulate with a tibial component formed of an organic polymer or polymer-based composite.

20. A method of making a prosthesis as claimed in any one of the preceding claims which comprises exposing a surface region of a prosthesis body to an oxygen or a nitrogen-containing environment for a predetermined length of time.

## Patentansprüche

1. Eine Prothese mit einem Prothesenkörper-Teil, der Zirkonium oder eine Zirkoniumlegierung aufweist, wobei der Körperteil einen Oberflächenbereich hat, der ein Oxid oder Nitrid des Zirkoniums aufweist, und die Sauerstoff- oder Stickstoff-Konzentration im Oberflächenbereich von einem Maximum an der äußeren Oberfläche zu einem Minimum im inneren Teil des Bereiches abnimmt.

2. Eine Prothese nach Anspruch 1, bei welcher der Oberflächenbereich des Prothesenkörper-Teils eine Tragfläche und ausgebildet ist, um an einen anderen Prothesenkörper anzugreifen.

3. Eine Prothese gemäß Anspruch 1 oder Anspruch 2, bei welcher der Prothesenkörper-Teil ausgebildet ist, um in Körpergewebe einzudringen und einzugreifen.

4. Eine Prothese nach Anspruch 3, bei welcher der Prothesenkörper-Teil eine Schraube ist.

5. Eine Prothese nach Anspruch 1 oder Anspruch 2, bei welcher der Prothesenkörper-Teil ausgebildet ist, um sich mit dem Körpergewebe oder einem anderen Teil einer Prothese zu verbinden.

6. Eine Prothese nach Anspruch 5, bei welcher der Prothesenkörper-Teil eine Knochenplatte ist.

7. Eine Prothese nach einem der vorhergehenden Ansprüche, bei welcher der Prothesenkörper-Teil eine modulare Komponente ist.

8. Eine Prothese zur Implantation in einem Patienten mit
a) einem Prothesenkörper, der aus Zirkonium oder einer Zirkoniumlegierung hergestellt ist, von dem wenigstens ein Teil ausgebildet ist, um in Körpergewebe einzudringen und einzugreifen, und
b) einer dünnen Beschichtung, die schwarzblaues oder schwarzes Zirkoniumoxid oder Zirkoniumnitrid direkt auf wenigstens dem Teil des Prothesenkörpers aufweist, der ausgebildet ist, um in Körpergewebe einzudringen und einzugreifen, um Freisetzung von Metallionen aus dem beschichteten Teil des Prothesenkörpers zu verhindern.

9. Eine Prothese nach einem der vorhergehenden Ansprüche, bei welcher der Körper-Teil weiterhin eine unregelmäßige Oberflächenstruktur aufweist, die ausgebildet ist, um Gewebeeinwachsungen an dem Teil des Prothesenkörpers aufzunehmen, der ausgebildet ist, um in Körpergewebe einzudringen und einzugreifen.

10. Eine Prothese nach Anspruch 9, bei welcher die unregelmäßige Oberflächenstruktur von Perlen aus Zirkonium oder einer Zirkoniumlegierrung gebildet wird, die mit der äußeren Oberfläche des Prothesenkörpers verbunden sind.

11. Eine Prothese nach Anspruch 9, bei welcher die unregelmäßige Oberflächenstruktur von einem Drahtgeflecht aus Zirkonium oder einer Zirkoniumlegierung gebildet wird, das mit der äußeren Oberfläche des Prothesenkörpers verbunden ist.

12. Eine Prothese nach einem der vorhergehenden Ansprüche, bei welcher sich der Oberflächenbereich über die gesamte Prothese ausdehnt.

13. Eine Prothese nach einem der vorhergehenden Ansprüche, bei welcher der Oberflächenbereich eine Dicke von weniger als 10 µm hat.

14. Eine Prothese zur Implantation in einem Patienten mit
a) einem Prothesenkörper, der aus Zirkonium oder einer Zirkoniumlegierung hergestellt ist und einen Implantat-Teil zum Eindringen und Eingreifen in das Körpergewebe des Patienten einschließt,
b) einer Tragfläche am Prothesenkörper, die von einer Größe und Gestalt ist, um an einer Tragfläche eines anderen Prothesen-Teils anzugreifen, und
c) einer dünnen Beschichtung aus Zirkoniumoxid direkt auf wenigstens der Tragfläche des Prothesenkörpers, um die Tragfläche inert und gegen Abnutzung widerstandsfähiger zu machen und mit einem niedrigeren Reibungskoeffizienten zu versehen.

15. Eine Prothese zur Implantation in einem Patienten mit
a) einem Prothesenkörper, der aus Zirkonium oder einer Zirkoniumlegierung hergestellt ist und einen Implantat-Teil zum Eindringen und Eingreifen in das Körpergewebe des Patienten einschließt,
b) einer Tagfläche am Prothesenkörper, die von einer Größe und Gestalt ist, um an der Tragfläche eines anderen Prothesen-Teils anzugreifen, und
c) einer dünnen Beschichtung aus Zirkoniumnitrid direkt auf wenigstens der Tragfläche des Prothesenkörpers, um die Tragfläche inert und gegen Abnutzung widerstandsfähig zu machen und mit einem niedrigeren Reibkoeffizienten zu versehen.

16. Eine Prothese nach einem der vorhergehenden Ansprüche, bei welcher der Prothesenkörper ein Teil einer Hüft-Prothese ist, die ausgebildet ist, um in einem Oberschenkelknochen implantiert zu werden.

17. Eine Prothese nach einem der Ansprüche 1 bis 11, bei welcher die Tragfläche des Prothesenkörpers einen Kopf-Teil aufweist, der ausgebildet ist, um mit einer Hüftgelenkpfanne ein Gelenk zu bilden, , die aus einem organischen Polymer oder einem Composite auf Polymerbasis hergestellt ist.

18. Eine Prothese nach einem der Ansprüche 1 bis 11, bei welcher der Prothesenkörper der Teil einer Knie-Prothese ist, die ausgebildet ist, um in einen Oberschenkelknochen oder einem Schienbeinknochen implantiert zu werden.

19. Eine Prothese nach einem der Ansprüche 1 bis 11, bei welcher die Tragfläche des Prothesenkörpers wenigstens ein Gelenkkopf-Teil ist, der ausgebildet ist, um mit einer Schienbein-Komponente ein Gelenk zu bilden, die aus einem organischen Polymer oder einem Composite auf Polymerbasis hergestellt ist.

20. Ein Verfahren zur Herstellung einer Prothese wie sie in einem der vorhergehenden Ansprüche beansprucht wird, bei welchem ein Oberflächenbereich des Prothesenkörpers einer Sauerstoff oder Stickstoff enthaltenden Umgebung für eine vorbestimmte Zeitdauer ausgesetzt wird.

## Revendications

1. Prothèse ayant un corps de prothèse comprenant du zirconium ou un alliage de zirconium, le corps ayant une région de surface comprenant un oxyde ou un nitrure de zirconium, dans laquelle la concentration en oxygène ou en azote dans la région de surface diminue d'une valeur maximale, à l'endroit de sa surface extérieure, à une valeur minimale à l'intérieur de ladite région.

2. Prothèse suivant la revendication 1, dans laquelle la région de surface du corps de prothèse est une surface de portée et elle est prévue pour venir en contact avec un autre corps de prothèse.

3. Prothèse suivant la revendication 1 ou la revendication 2, dans laquelle le corps de prothèse est prévu pour pénétrer et s'engager dans un tissu corporel.

4. Prothèse suivant la revendication 3, dans laquelle le corps de prothèse est une vis .

5. Prothèse suivant la revendication 1 ou la revendication 2, dans laquelle le corps de prothèse est prévu pour être attaché à un tissu corporel ou à une autre partie.

6. Prothèse suivant la revendication 5, dans laquelle le corps de prothèse est une plaque d'os.

7. Prothèse suivant une quelconque des revendications précédentes, dans laquelle le corps de prothese est un composant modulaire.

8. Prothèse pour implantation dans un patient, comprenant :
a) un corps de prothèse formé de zirconium ou d'alliage de zirconium, dont au moins une partie est prévue pour pénétrer et s'engager dans des tissus corporels, et
b) un revêtement mince comportant de l'oxyde de zirconium bleu-noir ou noir,ou du nitrure de zirconium, directement sur au moins la partie du corps de prothèse prévue pour pénétrer et s'engager dans les tissus corporels, afin d'empêcher la libération d'ions métalliques de la partie revêtue du corps de prothèse.

9. Prothèse suivant une quelconque des revendications précédentes,dans laquelle le corps comprend en outre une structure de surface irrégulière prévue pour permettre la croissance du tissu dans cette structure, sur la partie du corps de prothèse destinée à pénétrer et s'engager dans un tissu corporel.

10. Prothèse suivant la revendication 9, dans laquelle la structure de surface irrégulière est formée de perles de zirconium ou d'alliage de zirconium fixées à la surface extérieure du corps de prothèse.

11. Prothèse suivant la revendication 9, dans laquelle la structure de surface irrégulière est formée d'un grillage en zirconium ou alliage de zirconium fixé à la surface extérieure du corps de prothèse.

12. Prothèse suivant une quelconque des revendications précédentes, dans laquelle ladite région de surface s'étend sur toute la prothèse.

13. Prothèse suivant une quelconque des revendications précédentes,dans laquelle la région de surface a une épaisseur inférieure à 10 µm.

14. Prothèse pour implantation dans un patient comprenant :
a) un corps de prothèse en zirconium ou alliage de zirconium, incluant une partie d'implant pour pénétrer et s'engager dans des tissus corporels du patient ;
b) une surface de portée sur le corps de prothèse, la surface de portée étant dimensionnée et configurée de manière à venir en contact avec une surface de portée sur une autre partie de prothèse ; et
c) un revêtement mince d'oxyde de zirconium, directement sur au moins la surface de portée du corps de prothèse afin de rendre la surface de portée inerte, plus résistante à l'usure et de coefficient de frottement plus faible.

15. Prothèse pour implantation dans un patient, comprenant :
(a) un corps de prothèse en zirconium ou alliage de zirconium , incluant une partie d'implant pour pénétrer et s'engager dans des tissus corporels du patient;
(b) une surface de portée sur le corps de prothèse, la surface de portée étant dimensionnée et configurée de manière à venir en contact avec une surface de portée sur une autre partie de prothèse ; et
(c) un revêtement mince de nitrure de zirconium directement sur au moins la surface de portée du corps de prothèse, pour rendre la surface de portée inerte, plus résistante à l'usure et de coefficient de frottement plus faible.

16. Prothèse suivant une quelconque des revendications précédentes, dans laquelle le corps de prothèse est la partie d'une prothèse de hanche prévue pour être implantée dans un fémur.

17. Prothèse suivant une quelconque des revendications 1 à 11, dans laquelle la surface de portée du corps de prothèse comprend une partie de tête prévue pour s'articuler avec une coupelle acétabulaire formée d'un polymère organique ou d'un composite à base de polymère.

18. Prothèse suivant une quelconque des revendications 1 à 11, dans laquelle le corps de prothèse est la partie d'une prothèse de genou prévue pour être implantée dans un fémur ou un tibia.

19. Prothèse suivant une quelconque des revendications 1 à 11, dans laquelle la surface de portée du corps de prothèse est au moins une partie de condyle prévue pour s'articuler avec un composant tibial formé d'un polymère organique ou d'un composite à base de polymère.

20. Procédé de fabrication d'une prothèse suivant une quelconque des revendications précédentes, qui comprend l'exposition d'une région de surface d'un corps de prothèse à une atmosphère contenant de l'oxygène ou de l'azote, pendant un temps prédéterminé.
